# EUROPEAN PATENT APPLICATION

(11) **EP 3 741 398 A1**
(43) Date of publication of application: **25.11.2020**
(21) Application number: 19175374.8
(22) Date of filing: 20.05.2019
(51) Int. Cl.: A61L 27/28, A61L 27/36, A61L 27/54

(54) **METHOD FOR COATING A MEDICAL DEVICE AND COATED MEDICAL DEVICE**

(71) Applicant: Medizinische Hochschule Hannover, 30625 Hannover (DE)
(72) Inventor: STIESCH, Meike, 30177 Hannover (DE); STUMPP, Sascha Nico, 30629 Hannover (DE); EISENBURGER, Michael, 30966 Hemmingen (DE); RAHIM, Muhammad Imran, 30629 Hannover (DE)
(74) Representative: Gramm, Lins & Partner Patent- und Rechtsanwälte PartGmbB

(57) **Abstract**

The present invention relates in a first aspect to a method for coating a medical device suitable for implantation into an individual or for application on skin or mucosal tissue of an individual. Said method comprises the steps of applying to at least a portion of the surface of said device a coating layer whereby said coating layer comprises commensal microorganisms, like commensal bacteria, to form a biofilm on the at least portion of the surface of said medical device, further comprising the step of drying the biofilm coated on the surface of said medical device whereby the commensal microorganisms are eventually killed in case they were not applied as killed microorganisms in the step above, for obtaining a medical device having at least a portion of its surface coated with non-living commensal microorganisms. In a further aspect, the coated medical devices obtainable by the method according to the present invention are provided. The coated medical devices according to the present invention are particularly useful in applications being mucosal tissue or a skin of an individual, like for use as an implant for dental use in the oral cavity. Finally, the present invention relates to the use of commensal bacteria like *Streptococcus oralis* for coating a medical device suitable for use as an implant into an individual or for application on skin or mucosal tissue of an individual.

## Description

The present invention relates in a first aspect to a method for coating a medical device suitable for implantation into an individual or for application on the skin or the mucosal tissue of an individual. Said method comprises the steps of applying to at least a portion of the surface of said device a coating layer whereby said coating layer comprises commensal microorganisms, like commensal bacteria, to form a biofilm on the at least portion of the surface of said medical device, further comprising the step of drying the biofilm coated on the surface of said medical device whereby the commensal microorganisms are eventually killed in case they were not applied as killed microorganisms in the step above, for obtaining a medical device having at least a portion of its surface coated with non-living commensal microorganisms. In a further aspect, the coated medical devices obtainable by the method according to the present invention are provided. The coated medical devices according to the present invention are particularly useful in applications being mucosal tissue, bones or skin of an individual, like for use as medical implant in the oral cavity, orthopedic implant, mesh-like implant and cardiovascular implant. Finally, the present invention relates to the use of commensal bacteria like *Streptococcus oralis* for coating a medical device suitable for use as an implant into an individual or for application on skin or mucosal tissue of an individual.

### Prior art

Dental implants reinstate oral functions in partial (loss of some teeth) or complete edentulous (loss of all teeth) patients. A major challenge towards consistent functioning of dental implants remains biofilm formation, by infectious bacteria on their surfaces. Biofilm infections are highly inflammatory, potentially destroy host tissue (peri-implantitis) and are painful for patients. It is well documented that initial bacterial adhesion is a critical step towards invasion and biofilm formation on dental implants. These interpretations have led to detailed studies and counter-measures that interfere with the process of bacterial attachment by employing diverse methods including receptor analogues, mucopolysaccharides, vaccines against bacterial fimbriae, antimicrobial coatings, bacterial repellants surfaces and the adhesion of competitive bacteria. It is submitted that by preventing adhesion of pathogens on a surface, the infection can be effectively prevented. However, bacteria organized in biofilms have evolved numerous mechanisms to escape or resist host immune responses and successfully colonize within the host, including fast multiplication, rapid communication through quorum sensing, expression of various adhesins, use of nonspecific adhesion mechanisms (hydrophobic and electrostatic interactions) and formation of protective capsules. Therefore, there is an ongoing need to effectively combat pathogenic infections by methods being safe for the patients' health.

Dental implants are surgical components that interface with jaw or skull bones and support dental prosthesis. They are surgically implanted in the jaw bone at the place of a missing tooth. Their functioning and survival is based on successful osseointegration, a process comprising bone modelling and remodeling dependent on osteoblastic and osteoclastic activities, resulting both in a structural and functional connection between the implant and the bone. Titanium is the most common choice of materials for dental implants due to its excellent properties including biocompatibility, resistance to corrosion and mechanical resistance. After implantation, titanium implants are at risk of failure due to adverse foreign body response preventing direct contact between the implant and the bone despite the above characteristics and the presumed inertness of the material. At a later time point, implants are at risk of periimplant infections leading to bone resorption which ultimately can result in implant loss. One of the most important paradigm shifts seen in the field of bone implants in recent years concerns osteoimmunology as a crucial factor in osseointegration that considers implants not as inert but as immunomodulatory material, affecting the overall success of osseointegration. Another critical factor that influences the success of osseointegration and that has been recognized as a major factor for implants survival is infection related to pathological biofilm formation on implant surfaces. For example, Derks J. et al., J. Dent. Res. 2016, 95(1):43-49 shows that peri-implantitis affects 45 % of patients with dental implants. For example, of the approximately 500 species of microorganisms residing in the human oral cavity, 25 belong to the *Streptococcus genus*, which represents 20 % of the overall oral microflora. Some of these bacterial species are beneficial, while others are pathogenic for the human host. For example, *Streptococcus mutans* is strongly associated with dental caries formation. Interestingly, only half of these 500 species have been isolated and grown in culture.

It has been shown that microorganisms that live on biological or artificial surfaces exposed to air or liquids create complex communities known as biofilms. Biofilms can be composed of multiple bacterial species, non-randomly distributed in extracellular polymeric substances including polysaccharides, proteins, lipids and DNA. In the human body, biofilms can be found on the teeth where they form dental plaque.

*Streptococci* are among the initial colonizers to attach and form biofilm on the surface of a tooth or a dental implant in a symbiotic capacity. These bacteria are non-pathogenic per se, and can have a protective role by preventing the adhesion of pathogenic species. However, changes in the oral environment can promote association and attachment of pathogenic bacterial species that shifts the composition of the biofilm. This newly evolved biofilm can lead to dental caries or parodontitis. Some of the bacterial species often associated with tooth and gum diseases are *Porphyromonas gingivalis* and *Treponema denticola.* Interestingly, biofilm formation on teeth and dental implants follows a very similar sequence in humans with *Streptococcus oralis* being among the first species to attach to the empty surface.

Commensal bacteria are diverse microbial species which perform beneficial activities for human health. Recent scientific advances have evidenced an increase in the application of probiotics (live microorganisms which promote health if consumed in specific amount) as novel therapeutic strategy to counter pathogenic bacteria and to support the activities of host immune system. Human oral microbiome comprises of diverse bacterial species most of which are commensals and protect natural tissue or implants from the colonization of pathogens. Additionally, commensal bacteria support host immune mechanism and maintain a homeostatic balance between immune cells. However, pathogenic situations may arise in the oral cavity which can shift the balance towards higher colonization of infectious anaerobic oral pathogens. These pathogenic bacteria make infectious biofilms on dental implants and trigger robust host inflammatory reactions which culminate in tissue destruction and bone loss. Classical methods of treating biofilm infections through antibiotics are becoming relatively less efficacious thereby reliance on antibiotics needs to be minimized and novel therapeutic approaches are essentially required to counter adhesion of infectious pathogens. In this perspective, probiotics particularly those based on *Streptococci* have emerged as promising approach to counter bacterial infections and improve oral health. *Streptococcus salivarius* as being part of healthy oral microflora have displayed promising results as probiotics to treat pharyngeal mucosal infection, see Guglielmetti S. et al., Applied and Environmental Microbiology, 2010;76,3948-58. *Streptococcus oralis, Streptococcus uberis* and *Streptococcus rattus* as probiotics were also efficacious against infectious *Prevotella intermedia.* Several other studies support that probiotics improve oral health. Regular intake of probiotic *Lactobacillus* and *Bifidobacterium* was protective against cariogenic and plaque forming bacteria, e.g. Näse, L. et al., Caries research 2001;35:412-20.

Further, reduction in gingival inflammation and number of infectious biofilm forming *P. gingivalis* was observed by the use of probiotic *Lactobacillus.* Similarly, bacteriocins produced from *Streptococcus salivarius* were used as probiotics to treat plaque as a representative of oral biofilm in children and emerged as novel antimicrobial agents. Further, a use of intestinal probiotics *Lactobacillus rhamnosus GG, Lactobacillus reuteri* and *Bifidobacterium* effectively reduce the population of *S*. *mutans* and dental caries. Exopolysaccharide (EPS) extracted from *Lactobacillus plantarum* was effective to reduce infections caused by *Staphylococcus aureus, Listeria mono-cytogenes, Pseudomonas aeruginosa,* and *Salmonella typhimurium.*

Most of the studies performed so far have been focused on the application of probiotics (live healthy bacteria) to treat infections apart from a recent study that reported live as well as heat killed commensal *Lactobacillus* species were effective against oral biofilm forming pathogens, Mahdhi A. et al., Microbial Pathogenesis 2017, 109:214-20. This study identified heat killed commensal bacteria as effective strategy to counter infections. As described therein, the present oral biofilm forming pathogens are treated with the live as well as heat killed commensal microbacillus species. A prophylactic application is not described. Further, it is described that *Streptococcus oralis* in combination with *Streptococcus salivarius* has been successfully used to treat acute otitis media, La Mantia I. et al., International Journal of General Medicine 2017;10:171-5.

Some of the proposed solutions to solve the described problems, namely, infection after implantation, are based on introducing implant surface modifications, like sandblasting, acid etching etc. Said surface modifications should facilitate osteoblast attachment to the implant. Others focus on coatings that would improve osseointegration and reduce biofilm formation. The majority of such coatings are based on inorganic materials, like calcium phosphate, bioactive glass coating etc. that can additionally incorporate bioactive substances like growth factors and cytokines, to induce bone formation and reduce bone resorption or antimicrobial compounds such as antibiotics, antimicrobial peptides, metallic ions and nanoparticles to reduce biofilm formation, a review is given in Ana Civantos et al., ACS Biomater. Sci. Eng., 2017, 3(7), 1245-61.

Additionally, organic coatings based on synthetic or natural molecules including polyphosphacenes, polyehtylenglycol (PEG) and extracellular matrix proteins have also been tested. Although some of the aforementioned surface modifications and coatings provide limited protection against biofilm formation as outlined in Subramani K. et al., Int. J Oral Maxillofac Implants, 2009, 24(4):616,26, they are based on unselective bactericides that kill normal commensal microflora and might induce antibiotic resistance (in the case of antibiotic containing coatings).

It is clear from the above that innovative therapeutic techniques effective against pathogenic infections and being safe for the patients' health are required.

### Brief description of the present invention

In a first aspect, the present invention relates to a method for coating a medical device suitable for implantation into an individual or for application on skin or mucosal tissue of an individual comprising the steps of:
- applying to at least a portion of the surface of said device a coating layer whereby said coating layer comprises commensal microorganisms, like commensal bacteria, to form a biofilm on the at least portion of the surface of said medical device
- drying the biofilm coated on the surface of said medical device, eventually killing the commensal microorganism, for obtaining a medical device having at least a portion of its surface coated with non-living commensal microorganisms.

In particular, an aspect according to the present invention is a method for coating a medical device suitable for implantation into an individual or for application on skin or mucosal tissue of an individual wherein said implant is for implantation in the oral cavity and the commensal microorganisms are of the genus *Streptococcus,* in particular, of the species *Streptococcus oralis.*

In a further aspect, the present invention provides a coated medical device suitable for implantation into an individual for application on skin or mucosal tissue of an individual obtainable by a method according to the present invention. The coated medical device is suitable as implant for dental use, in particular, in the oral cavity.

In a further aspect, the use of commensal microorganisms, in particular, commensal bacteria for coating a medical device suitable for use as an implant in an individual or application on skin or mucosal tissue of an individual, is described.

### Brief description of the drawings

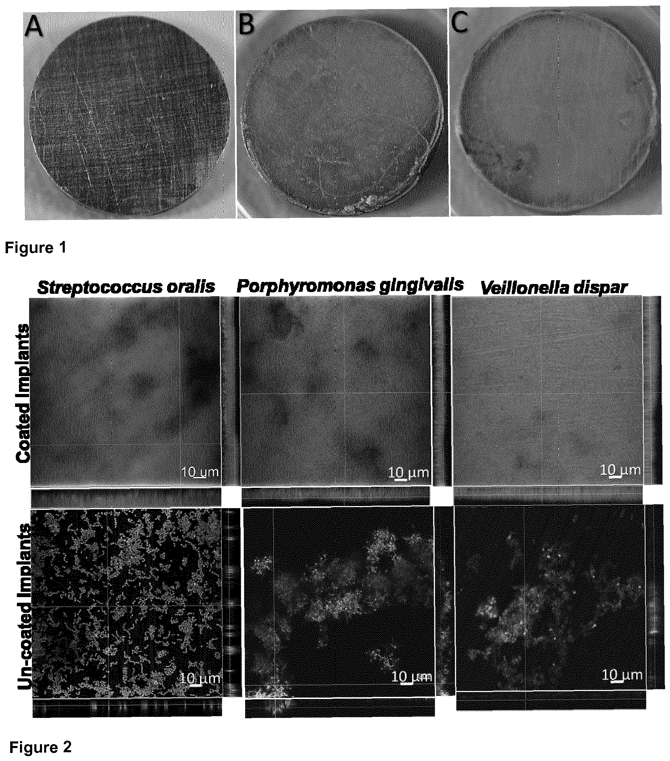

Figure 1 optical imaging of coated implants: titanium implants without coatings (A), titanium implants immediately after coating with *S*. *oralis* (B), coated titanium after 24 hours of incubation in cell culture medium (C).
Figure 2 figure 2 shows heat mediated absorption of titanium implants with specific oral commensal bacteria preventing colonization of infectious oral pathogens. The upper row shows the data for implants coated with *Streptococcus oralis* and incubated for 48 hours with planktonic cultures of *Streptococcus oralis, Porphyromonas gingivalis* and *Veillonella dispar* as identified. No biofilm formation was determined. In contrast, as shown in the lower row, *Streptococcus oralis, Porphyromonas gingivalis* and *Veillonella dispar* form biofilms on the surface of uncoated titanium.

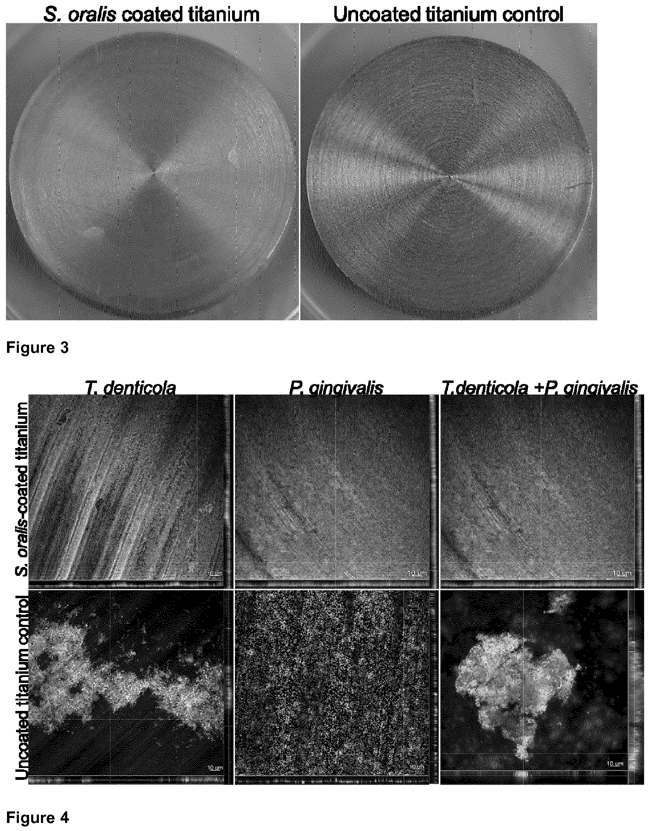

Figure 3 figure 3 identifies the surface morphology of titanium coated with heat killed *Streptococcus oralis* and uncoated control (right). The discs were incubated as described in example 2.
Figure 4 figure 4 shows titanium implants coated with heat killed *S*. *oralis* and then incubated with different pathogenic strains thereby inhibiting biofilm formation. *Treponema denticola* and *Porphyromonas gingivalis* alone or in combination were cultured with the titanium discs either coated with *S*. *oralis* or uncoated controls. Staining was effected as described using a fluorescent live/dead staining. Shown are confocal laser-scanning microscope images of *S*. *oralis* coated titanium in the upper row showing no biofilm formation while viable bacteria biofilms from *T*. *denticola, P. gingivalis* and combinations of both are shown in the lower row of uncoated titanium control.

### Detailed description of the present invention

In a first aspect, a method for coating a medical device suitable for implantation into an individual or for application on skin or mucosal tissue of an individual comprising the steps of:
- applying to at least a portion of the surface of said device a coating layer; whereby said coating layer comprises commensal microorganisms, like commensal bacteria, to form a biofilm on the at least portion of the surface of said medical device;
- drying the biofilm coated on the surface of said medical device, eventually killing the commensal microorganism, for obtaining a medical device having at least a portion of its surface coated with non-living commensal microorganisms;
is provided.

The present inventors recognized that coating of a medical device suitable for implantation, in particular, an implant coated beforehand with commensal microorganisms, thus, forming a biofilm on at least a portion of the surface of said medical device to be implanted, reduces the formation of pathogenic biofilms on implants besides preventing pathogenic bacterial attachment before. In addition, the commensal bacteria coated as biofilm on the medical device may modulate the immune system towards an anti-inflammatory response. The method according to the present invention allows to provide stable coatings on medical devices, in particular, surfaces of said medical devices including implants. In addition, the method represents a simple process using commensal microorganisms, in particular commensal microorganisms present in the oral cavity in case of use in the oral cavity including *Streptococcus oralis.* In addition, since the commensal microorganisms, like the commensal bacteria are coated as non-living commensal microorganisms after the drying process, no harm for the implantation environment where the medical device is implanted is expected.

As used herein, the term "comprise" or "comprising" as well as the term "contain" and "containing" include the embodiments of "consist of" or "consisting of".

As used herein, the term "medical device" includes all kind of medical devices suitable for implantation into an individual. The material of said medical device can be selected from any material suitable for implantation into an individual or for application on skin or mucosal tissue of an individual. Typically, the materials of the medical device are inert materials including metal and polymers.

The medical device may be a device for transient or permanent residence in the individual or on the skin or mucosal tissue of said individual e.g. made of titanium and its alloys, zirconia, stainless steel. The material may be a biodegradable material including suitable biodegradable materials on the basis of metals, e.g. magnesium based biodegradable material. Alternatively or in combination, biodegradable biopolymers may be used, e.g. polylactic acid composite.

In an embodiment of the present invention, the material of the medical device is a metal or an alloy. For example, the metal or alloy consisting of titanium or is a titanium containing alloy. In another embodiment, the medical device is or contains magnesium-based biodegradable material as described in the art.

In an embodiment of the present invention, the medical device is an implant for dental use. Said implant for dental use include an abutment, subperiosteal implants, transosteal implants, endosteal implants and ramus frame implants and other maxillofacial implants to reconstruct the bone. In an embodiment, the medical device is or contains the metal, like titanium, for dental use in the oral cavity. Further, in case of using said medical device being in contact with mucosal tissue or skin of an individual, said medical device may be urinary catheters, all types of intravenous catheters, intravaginal and intraintestinal devices (like stomach tubes, sigmoidoscopies, colonoscopies, gastroscopes) and external prosthesis.

The medical device according to the present invention does not require any pretreatment to allow coating with the commensal bacteria. That is, the medical device after normal cleaning, optionally including sterilization, is coated with the commensal microorganism as described herein. In particular, the method according to the present invention represents a cheap and easy method without any complicated tools required to achieve the coating. Rather, the drying, e.g. by heat mediation, is a simple method which can be applied for coating said medical device accordingly.

Moreover, the use of the commensal microorganisms according to the present invention represents a coating which is non-toxic to the individual receiving the implant or medical device. The coated commensal microorganisms are eventually killed, e.g. they are already applied as dead commensal microorganisms or are killed when heating the medical device. In an embodiment of the present invention, the medical device is placed on a heating device and heated before applying the commensal microorganisms to be coated on its surface. When the medical device is heated, the coating material, namely, the suspension of commensal microorganisms is applied to the surface of the heated device, e.g. by adding drops of suitable amounts onto the surface of the medical device. The drops including the bacteria are heated, thus, eventually killing the commensal microorganisms while the medical device maintains on the heating device.

In an embodiment of the present invention, the step of applying a coating layer and drying of the coating with the commensal microorganisms is repeated at least once, like at least twice, e.g. at least three times, four times, five times, six times, seven times, eight times, nine times, 20 times or multiple times. Repeating at least once the application of the coating material on the device improves the coating layer formed. In particular, the stability of the coating is improved when applying the coating layer at least twice or multiple times.

In an embodiment, the medical device to be coated is heated, like heating on a heating device, as mentioned above. The temperature of the heating device is at least 50°C to about 90°C. For example, the temperature of the device is in between 60°C to 90°C, like 65°C to 85°C, like 70°C to 80°C.

The suspension used for coating is typically a suspension of the commensal microorganisms in water or other suitable solvents. When adding the material to the medical device, the material is dried, e.g. by heating. The skilled person is well-aware of suitable means for effecting drying, thus, obtaining a biofilm coated on the surface of said medical device. As noted before, said step of applying the suspension on the surface of the medical device may be repeated once or several times. Thus, a stable coating is obtained. Said coating is non-toxic for the individual. In addition, the coating with the commensal microorganisms, like the oral commensal microorganisms in case of medical devices for the oral cavity, would be biocompatible for the host tissue since the oral commensals have symbiotic relations and live in homeostasis with host immune cells. That is, the coating according to the present invention has benefits also with respect to the immune response and alteration of the immune response. Further, the use of the commensal microorganisms, for example oral microflora in case of medical devices for oral cavity, plays an important role in supporting host immune system. This technology would guide and augment the functionalities of host immune system.

Further, the commensal microorganisms, like the commensal bacteria according to the present invention, interfere with pathogenic bacteria which try to adhere to the medical device, in particular, try to form a biofilm on said medical device, thus keeping the pathogenic bacteria in free form and not in a biofilm thereby allowing easy killing by the host immune cells. Contrary to the mechanisms of action of antibiotics, factors that inhibit the adhesion generally do not kill infectious bacteria. Accordingly, through present technology chances of evolving antibacterial resistance among bacteria will be significantly reduced.

The commensal microorganisms in the coating step may be living commensal microorganisms. Alternatively, said commensal microorganisms to be coated are killed e.g. by heat in advance. In an embodiment, the coating is a suspension of living commensal microorganisms. Alternatively, a suspension of killed commensal microorganisms is applied.

The commensal microorganisms according to the present invention include commensal bacteria. The term "commensal microorganism" as used herein refers to all types of microorganisms living on or in an individual without causing a pathologic reaction. According to this definition the term commensal microorganisms covers all commensal bacteria and all other potential pathogens such as viruses and parasites.

In an embodiment of the present invention, the commensal microorganisms are commensal bacteria. For example, the commensal bacteria are of the genus *Streptococcus.* In an embodiment, the commensal bacteria are of the species *Streptococcus oralis.*

As demonstrated in the example, the application of the heat killed commensal bacteria in form of a coating represents an effective strategy to counter infections. Since initial adhesion of pathogens is a vital step towards the formation of infectious biofilms on implant surfaces, the present inventors have devised a strategy to coat the medical device, like the dental implant surfaces with *S. oralis* through heat mediation process. As shown, this coating prevented initial adhesion of infectious biofilm forming pathogens. *Streptococcus oralis* as a member of the oral microflora exhibit the symbiotic relation with host immune system and is among the first bacteria to incur the implant material or teeth surfaces.

In an embodiment of the present invention, the method is a method wherein the coating is a complete coating of the outer surface of said medical device, in particular, medical devices for oral or dental use.

In another aspect, the present invention relates to a coated medical device suitable for implantation into an individual or for application on skin or mucosal tissue of an individual obtainable by a method according to the present invention. As mentioned above, the coated medical device according to the present invention with the coating layer identified herein, displays superior properties compared to other coatings including improved stability as well as non-toxicity against the individual receiving the medical device.

In another embodiment, the coated medical device according to the present invention is an implant for dental use, like an abutment, subperiosteal implants, transosteal implants, endosteal implants and ramus frame implants. In another embodiment, the medical device suitable for application on skin or mucosal tissue of an individual is e.g. urinary catheters, intravaginal and intraintestinal devices (stomach tubes, sigmoidoscopies, colonoscopies, gastroscopes) and external prosthesis. The coated medical device is superior with respect to avoiding infection with pathogenic microorganisms when applied as an implant or when used in applications being in contact with mucosal tissue or skin of an individual.

In an embodiment, the coated medical device according to the present invention is composed of or contains a metal, in particular, titanium, for dental use in the oral cavity. In particular, the coated medical device according to the present invention does not require specific pretreatment of the surface of the metal or alloy, in particular, metal or alloy containing titanium. In a preferred embodiment, the coated medical device according to the present invention is an implant for dental use, in particular for use in the oral cavity composed or containing titanium whereby the outer surface of said medical device is coated partially or completely with a biofilm comprising commensal microorganisms as described herein. This coated medical device has various benefits over the prior art implants. As described above, the biofilm of the commensal microorganisms, e.g. of the genus *Streptococcus* and, in particular, of the species *Streptococcus oralis* is beneficial for the immune system of the host as well as acting against pathogenic bacteria.

In a further embodiment, the use of commensal microorganisms, in particular, commensal bacteria for coating a medical device suitable for use as an implant into an individual or for application on skin or mucosal tissue of an individual is described.

In particular, the use according to the present invention is a used of said commensal microorganisms, like commensal bacteria for coating the surface of a medical device for application into the oral cavity of an individual.

In an embodiment, the use according to the present invention is a use wherein the commensal bacteria, in particular, *Streptococcus oralis*, is applied as living bacteria on the surface of said medical device when producing the same and killing said bacteria after coating, in particular, when the medical device to be coated is heated on a heating device or similar devices.

In a preferred embodiment, the use according to the present invention is a use of commensal bacteria comprising *Streptococcus oralis.*

The coated medical device according to the present invention is composed of suitable materials. As described above, suitable materials include metals and polymers, like biocompatible and biodegradable metals or alloys and polymers. The coated medical device may be for transient or permanent use in said individual.

The present invention will be described further by way of examples without limiting the same thereto.

### Example 1

### Preparation of the coating dispersion:

*Streptococcus oralis* (ATCC 9811, American Type Culture Collection, Manassas, USA) were cultured overnight in TSB (Trypton Soya Broth) at 37°C under shaking speed of 200 RPM. Overnight cultures were then adjusted to optical density of OD₆₀₀=1.5 and then centrifuged at 4000xg for 15 minutes. After centrifugation, supernatant was discarded and bacterial pellet was resuspended in Milli-Q water and centrifuged again at 4000xg for 15 minutes. The supernatant was discarded and bacterial pellet was resuspended in Milli-Q water for obtaining suspension for implant coating.

### Coating of titanium disc

Sterilized titanium discs 9 mm in diameter and 3 mm in thickness were placed on a hot plate adjusted to 75°C. 50 µl of bacterial suspension was then added on titanium disc, liquid was allowed to evaporate and when the surface of titanium became dry, 50 µl of bacterial suspension was added again. This procedure was repeated until a total bacterial suspension of 1 ml (OD₆₀₀=1.5) was coated on a single titanium disc. To get homogenous coating layer, bacterial suspension was regularly vortexed during the whole coating process.

In figure 1B disc obtained according to the present invention are shown. In addition, a titanium disc with no coating is shown in figure 1A.

### Titanium discs coated with Streptococcus oralis resist the adhesion of oral biofilm forming pathogens

First, to check the stability of coatings, freshly coated titanium discs as shown in figure 1B were incubated in cell culture medium for 48 hours at 37°C with 5 % CO₂ and then optically imaged. Even after incubation, bacterial coatings were clearly visible on titanium surfaces thereby confirming that coatings were stable and resistant towards a direct exposure to liquid medium, see figure 1C.

### Antimicrobial assays with coated and uncoated titanium discs

For antimicrobial assays, coated and for comparison uncoated (each in triplicates) titanium discs were incubated with fresh cultures of *Streptococcus oralis, Porphyromonas gingivalis* and *Veillonella dispar.* Discs were incubated with 500 µl of *Streptococcus oralis* adjusted initially to OD₆₀₀=0.05 in TSB (Oxoid Limited, Hamp-shire, UK) supplemented with 10 % yeast extract (Carl Roth GmbH + Co. KG, Karlsruhe, Germany) and 50 mM glucose (Carl Roth GmbH + Co. KG, Karlsruhe, Germany) at 37°C in the presence of 5 % CO₂. In parallel, discs were incubated with *Veillonella dispar* and *Porphyromonas gingivalis* (both at OD₆₀₀=0.05) in Brain heart infusion (BHI) medium supplemented with vitamin K at 37°C under anaerobic conditions. After 48 hours of incubation under static conditions, old medium was carefully removed and specimens were washed once with phosphate-buffered saline (PBS). Both coated as well as uncoated specimens were then treated with fluorescent LIVE/DEAD BacLight Bacterial Viability Kit (Life Technologies, Darmstadt, Germany). Both live/dead fluorescent dyes diluted at 1:1000 in PBS were applied and specimens were incubated in darkness for 15 minutes at room temperature. Biofilms on samples were then fixed with 2.5 % glutardialdehyde in PBS at 4°C for 15 minutes. All samples were then imaged by confocal laser-scanning microscopy (Leica TCS SP2, Leica Microsystems, Mannheim, Germany). The images were further possessed in the Imaris x 64 6.2.1 software package (Bitplane AG, Zurich, Switzerland). Coated and for comparison uncoated titanium discs were incubated with biofilm forming pathogens under standard laboratory conditions. As shown in figure 2, upper row the titanium implants coated with *S*. *oralis* inhibited the adhesion and subsequently biofilm formation by fresh *Streptococcus oralis, Veillonella dispar* and infectious *Porphyromonas gingivalis.* In contrast, uncoated titanium discs biofilm formation occurs by the bacteria accordingly, see figure 2, lower row.

### Titanium discs pre coated with Streptococcus oralis are compatible with human gingival fibroblasts

In the following, cellular viability of mammalian cells is tested. Biocompatibility of titanium coated with *S*. *oralis* was investigated with human gingival fibroblasts by employing a fluorometric CellTiter-Blue assay, which measures cellular reduction capacity through resazurin color change as described in the art. Gingival fibroblasts were cultivated together with titanium discs coated by commensal *S*. *oralis* and for comparison with uncoated titanium discs. Cells were cultured in Dulbecco's modified Eagle medium (Biochrom AG, Berlin, Germany) supplemented with 10 % fetal bovine serum (PAN-BIOTECH GmbH, Aidenbach, Germany), 100 U/ml penicillin, and 100 µg/ml streptomycin (Biochrom AG, Berlin, Germany) in a humidified cell culture incubated at 37°C in the presence of 5 % CO₂. After 24, 48 and 72 hours of incubation, existing DMEM was removed from cells and CellTiter-Blue reagent (Promega, Mannheim Germany) was added followed by 4 hours incubation at 37°C. After incubation, CellTiter-Blue reagent was collected from respective cells and its fluorescence was measured by using a multiwall plate reader (λₑₓ = 530 nm, λₑₘ = 590 nm, Synergy 2, BioTek). Gingival fibroblasts remain compatible with *S*. *oralis* coated titanium and their viability was similar to the cells cultured on uncoated titanium implants (data not shown). Overall, these findings are supportive for further application of *S*. *oralis* coatings in humans and animals.

### Example 2

### Coating with heat killed S. oralis

As described in example 1, titanium disc were coated with a suspension of heat killed *S*. *oralis.*

### Titanium implants coated with heat killed S. oralis inhibit biofilm formation Bacterial growth and coating

*Streptococcus oralis* (ATCC 9811, American Type Culture Collection, Manassas, USA) were overnight cultured at 37°C in TSB (Trypton Soya Broth) to optical density of 1.50 under shaking conditions. The cultures were then centrifuged at 4000xg for 15 minutes and bacterial pellet was resuspended in Milli-Q water and centrifuged for the second time at 4000xg for 15 minutes. The supernatant was then discarded and bacterial pellet was mixed in Milli-Q water and heated at 75°C for 30 minutes. Titanium were then coated with heat killed bacteria in way that 1 ml suspension of the heat killed bacteria was added on titanium disc and then incubated for 5 days at 37°C. This incubation triggered slow evaporation process and a gradual accumulation of heat killed bacteria on titanium discs. This process was adopted for all the coated titanium implants. As shown in figure 3, the surface morphology of the titanium coated with heat killed *Streptococcus oralis* was similar to the coating with the live *S*. *oralis.*

### Anti-biofilm assay

*In vitro* anti-biofilm properties of coatings were evaluated by incubating coated and for comparison uncoated titanium discs with *Porphyromonas gingivalis* and *Treponema denticola* cultures (each in triplicates). Titanium discs were incubated separately with 500 µm of *P. gingivalis* (OD₆₀₀=0.05) in Brain heart infusion (BHI) medium and *T. denticola* in new oral spirochete (NOS) medium at 37°C for 48 hours under anaerobic conditions. Side by side, coated and uncoated titanium were incubated with combination of *P. gingivalis* and *T. denticola* at indicated conditions.

After 48 hours, all specimens were washed with phosphate-buffered saline (PBS) and then treated with fluorescent LIVE/DEAD BacLight Bacterial Viability Kit (Life Technologies, Darmstadt, Germany) for 15 minutes at room temperature. All samples were then imaged by confocal laser-scanning microscopy (CLSM, Leica TCS SP2, Leica Microsystems, Mannheim, Germany). Acquired images were further processed in the Imaris x 64 6.2.1 software package (Bitplane AG, Zurich, Switzerland).

As demonstrated either slow evaporation but also heat-mediated coating results in a coating visible on the surface of titanium and establish suitable means for coating of medical devices, see figure 4, upper row coated titanium disc, lower row uncoated titanium disc. Both types of coatings inhibit biofilm formation by periodontal pathogens, *P. gingivalis* and *T. denticola.* Interestingly, anti-biofilm properties remain the same even when both periodontal pathogens were used in combination. These results validate the present invention by confirming the application of *S*. *oralis* as an example of commensal microorganisms, in particular, of the genus *Streptococcus,* coating either through heat mediation or slow evaporation is anti-biofilm active.

## Claims

1. A method for coating a medical device suitable for implantation into an individual or for application on skin or mucosal tissue of an individual comprising the steps of:
- applying to at least a portion of the surface of said device a coating layer whereby said coating layer comprises commensal microorganisms, like commensal bacteria, to form a biofilm on the at least portion of the surface of said medical device,
- drying the biofilm coated on the surface of said medical device, eventually killing the commensal microorganism, for obtaining a medical device having at least a portion of its surface coated with non-living commensal microorganisms.

2. A method for coating a medical device according to claim 1 wherein the step of applying a coating layer and drying of the coating with commensal microorganisms is repeated at least once, like at least twice, e.g. at least three times.

3. The method according to any one of the preceding claims wherein the commensal microorganisms in the coating layer step are living commensal microorganisms, in particular, wherein the coating layer is a suspension of living commensal microorganisms.

4. The method according to any one of the preceding claims whereby the medical device to be coated is heated, e.g. on a heating device, before applying the commensal microorganisms to be coated on its surface.

5. The method according to any one of the preceding claims wherein the surface of said medical device is metal or an alloy, like titanium or titanium containing alloy.

6. The method for coating a medical device according to any one of the preceding claims wherein the commensal microorganisms are of the genus *Streptococcus,* in particular, of the species *Streptococcus oralis.*

7. The method according to any one of the preceding claims wherein the coating is a complete coating of the outer surface of said medical device.

8. Coated medical device suitable for implantation into an individual or for application on skin or mucosal tissue of an individual obtainable by a method according to any one of claims 1 to 7.

9. The coated medical device according to claim 8 being an implant for dental use, like an abutment.

10. The coated medical device according to claim 8 for use in applications being in contact with mucosal tissue or skin of an individual.

11. The coated medical device according to any one of claims 8 to 10 composed or containing a metal, in particular, titanium, for dental use in the oral cavity.

12. A use of commensal microorganism, in particular, commensal bacteria, for coating a medical device suitable for use as an implant into an individual or for application on skin or mucosal tissue of an individual.

13. The use according to claim 12 for coating the surface of a medical device for application in the oral cavity of an individual.

14. The use according to claim 12 or 13 of commensal bacteria comprising *Streptococcus oralis.*

15. The use according to anyone of claims 12 to 14 wherein the commensal bacteria, in particular, *Streptococcus oralis,* are applied as living bacteria onto the surface of said medical device when producing the same and killing said bacteria after coating.
